# EUROPÄISCHE PATENTSCHRIFT

(11) **EP 2 383 568 B1**
(45) Veröffentlichungstag und Bekanntmachung des Hinweises auf die Patenterteilung: **04.04.2018**
(21) Anmeldenummer: 11163298.0
(22) Anmeldetag: 20.04.2011
(51) Int. Cl.: G01N 33/36, G01N 15/08

(54) **Vorrichtung und Verfahren zur Prüfung der Luftdurchlässigkeit von Textilien**
Method and device for testing the air permeability of textiles
Procédé et dispositif de contrôle de la perméabilité à l'air de textiles

(30) Priorität: 30.04.2010 DE 102010019178
(43) Veröffentlichungstag der Anmeldung: 02.11.2011
(73) Patentinhaber: Steinel GmbH, 33442 Herzebrock (DE)
(72) Erfinder: Kumpost, Roman, 46014, Liberec 14 (CZ)
(74) Vertreter: Patentanwälte Behrmann Wagner PartG mbB

(56) Entgegenhaltungen:
- EP-A1- 0 373 058
- DE-C1- 4 217 682
- US-A- 4 198 854
- US-A- 4 495 796
- US-A- 6 119 506
- US-B2- 6 843 106

## Beschreibung

### Stand der Technik

Die Erfindung betrifft eine Vorrichtung zur Prüfung der Luftdurchlässigkeit von Textilien nach dem Oberbegriff des Anspruchs 1.

Eine derartige Vorrichtung ist aus der DE 42 17 682 C1 bekannt. Sie dient zur laufenden Qualitätsüberwachung bzw. Prüfung der Luftdurchlässigkeit von Textilbahnen. Hierzu ist eine Prüfvorrichtung vorgesehen, die quer zu einer Textilbahn verschiebbar angeordnet ist.

Im Zusammenhang mit bestimmten Sportarten, beispielsweise dem Skispringen oder im Schwimmsport, bestehen Wettkampfvorschriften, die die beim Skispringen bzw. beim Schwimmen getragene Kleidung spezifizieren. So ist es beispielsweise beim Skispringen bekannt, dass die internationale Skisportvereinigung FIS Vorschriften bezüglich der Luftdurchlässigkeiten eines Skisprunganzugs vorschreibt. Gegenwärtig muss ein Skisprunganzug beispielsweise eine minimale Luftdurchlässigkeit von 40 Liter pro m² und Sekunde bei einem Messunterdruck von 10mm Wassersäule bei einem nicht gespannten Gewebe aufweisen. Die Prüfung einer derartigen Luftdurchlässigkeit lässt sich mit der eingangs erwähnten Vorrichtung bei der Produktion von Skianzügen beispielsweise feststellen bzw. überprüfen. Problematisch ist jedoch, dass derartige Überprüfungen auch im Rahmen des Wettkampfes vor Ort stattfinden müssen, um gegebenenfalls Manipulationen an den Skianzügen auszuschließen bzw. für gleiche Wettkampfbedingungen für die einzelnen Teilnehmer zu sorgen. Hierbei ist es insbesondere problematisch, dass sich die verschiedenen Wettkampfstätten an unterschiedlichen Orten, insbesondere auch auf unterschiedlichen Höhen über dem Meeresspiegel, befinden. Dadurch ist es schwierig, die angesprochene Überprüfung hinsichtlich des Grenzwertes zu gewährleisten bzw. sicherzustellen, da der Messort einen Einfluss auf das Messergebnis hat.

Aus der US 6,119,506 ist eine Vorrichtung nach dem Oberbegriff des Hauptanspruchs bekannt. Zum weiteren Stand der Technik wird verwiesen auf die US 4,198,854.

### Offenbarung der Erfindung

Ausgehend von dem dargestellten Stand der Technik liegt der Erfindung die Aufgabe zugrunde, eine Vorrichtung zur Überprüfung der Luftdurchlässigkeit von Sporttextilien nach dem Oberbegriff des Anspruchs 1 derart weiterzubilden, dass diese, unabhängig von dem geographischen Ort der Überprüfung der Textilprobe, stets zuverlässige Ergebnisse, insbesondere bezüglich eines vorgegebenen Grenzwertes, liefert. Diese Aufgabe wird bei einer Vorrichtung zur Überprüfung der Luftdurchlässigkeit von Sporttextilien mit den Merkmalen des Anspruchs 1 gelöst. Der Erfindung liegt dabei die Idee zugrunde, die Auswertemittel, die zur Berechnung der Luftdurchlässigkeit dienen, mit einer Kompensationseinrichtung zu koppeln, mittels der insbesondere die jeweils örtlich spezifischen Gegebenheiten bzw. Umgebungsbedingungen des Ortes der Überprüfung der Luftdurchlässigkeit der Textilien angepasst werden kann.

Vorteilhafte Weiterbildungen der erfindungsgemäßen Vorrichtung zur Prüfung der Luftdurchlässigkeit von Textilien sind in den Unteransprüchen angegeben.

Es ist dabei erfindungsgemäß vorgesehen, dass die Kompensationseinrichtung zumindest eine Einrichtung zur Erfassung des Absolutdrucks der Umgebungsluft aufweist. Die Erfindung macht sich dabei die Erkenntnis zunutze, dass bei der Berechnung der Luftdurchlässigkeit es erforderlich ist, nicht nur den Differenzdruck zwischen der Umgebungsluft und den Druck in der Messkammer zu berücksichtigen bzw. einzustellen, sondern darüber hinaus auch den Absolutdruck der Luft zu berücksichtigen, da dieser in die Dichteberechnung der Luft und damit auch in die Berechnung der Luftdurchlässigkeit der Textilprobe eingeht. Dieser Absolutdruck kann dann entweder automatisch berücksichtigt werden, ergänzend oder alternativ, mittels einer Anzeigeeinheit sowie manuell betätigbaren Einstellmitteln eine Betätigung durch eine Bedienperson ermöglichen.

Besonders vorteilhaft ist es weiterhin, wenn die Kompensationseinrichtung zusätzlich eine Einrichtung zur Erfassung der Temperatur der durch die Textilprobe der strömenden Luft aufweist. Dies ist deshalb sinnvoll, da auch die Temperatur der die Textilprobe durchströmenden Luft einen Einfluss auf die Berechnung der Luftdurchlässigkeit ausübt. Dabei ist insgesamt zu berücksichtigen, dass die zur Diskussion stehenden Sporttextilien, zum Beispiel Skianzüge, aus Gründen ihrer optimalen Leistungsfähigkeit oftmals sehr nah an dem zugelassenen Grenzwert ausgelegt sind, so dass es ansonsten ohne eine entsprechende Berücksichtigung der Parameter des Absolutdrucks der Umgebungsluft sowie der Temperatur der Luft zu einer Überschreitung des Grenzwertes und somit beispielsweise zu einer Disqualifikation des Wettkampfteilnehmers kommen kann. Daher ist es erwünscht, möglichst sämtliche Parameter, die die Luftdurchlässigkeit beeinflussen, möglichst genau und umfassend zu berücksichtigen, damit es nicht zu Falschmessungen kommt.

Um eine möglichst genaue Messung der Temperatur bzw. Messung der Luftdurchströmung durch die Textilprobe zu ermöglichen, wird darüber hinaus vorgeschlagen, dass die Einrichtung zur Erfassung der Temperatur unmittelbar vor der Einrichtung zur Erfassung des Luftvolumenstroms angeordnet ist. Dadurch werden der Messort für die Temperatur und der Messort für die Luftdurchströmung örtlich zusammengelegt, so dass besonders präzise Messungen möglich sind.

Besonders bevorzugt ist es weiterhin, wenn die Vorrichtung als tragbare, mobile Prüfeinrichtung mit einem vorzugsweise einteiligen, die Einrichtungen aufnehmenden Prüfstandskoffer ausgebildet ist, der gegebenenfalls mit einem Schutzelement zum Abdecken des Prüfstandskoffers verbindbar ist. Dadurch wird ein erfindungsgemäßer mobiler Einsatz der Vorrichtung an der jeweiligen Wettkampfstätte ermöglicht, ohne dass hierzu besondere Vorrichtungen bezüglich der Infrastruktur erforderlich sind.

Die Erfindung umfasst auch ein Verfahren zur Prüfung der Luftdurchlässigkeit von Sporttextilien, mit den in Anspruch 5 definierten Schritten. Hierbei ist es wesentlich, dass der Auswerteeinrichtung wenigstens eine zusätzliche Kompensationsgröße als Eingangswert zugeführt wird, wobei die wenigstens eine Kompensationsgröße der Absolutdruck der Umgebungsluft ist. Dabei ist es vorteilhaft, wenn der Messung der Luftdurchlässigkeit der Textilprobe ein automatischer Prüfablauf zur Überprüfung von Komponenten und Sensoren vorausgeht. Dadurch kann eine Fehlfunktion von Komponenten vor dem Testen der Textilprobe festgestellt werden, wobei durch den automatischen Ablauf die Bedienung der Vorrichtung wesentlich vereinfacht wird.

Besonders bevorzugt ist die Verwendung der Vorrichtung zum Testen von Skisprunganzügen und Schwimmanzügen.

Weitere Vorteile, Merkmale und Einzelheiten der Erfindung ergeben sich aus der nachfolgenden Beschreibung bevorzugter Ausführungsbeispiele sowie anhand der Zeichnungen.

Diese zeigen in:
- Fig. 1: eine perspektivische Ansicht auf eine Vorrichtung zur Prüfung der Luftdurchlässigkeit von Textilien,
- Fig. 2: eine schematische Darstellung des Aufbaus einer erfindungsgemäßen Vorrichtung zur Prüfung der Luftdurchlässigkeit von Textilien und
- Fig. 3: ein Flussablaufdiagramm zur Darstellung des Prüfablaufs bei der Prüfung der Luftdurchlässigkeit einer Textilprobe.

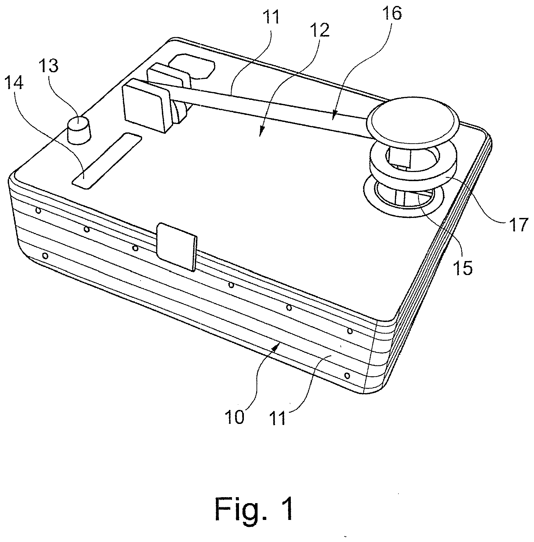

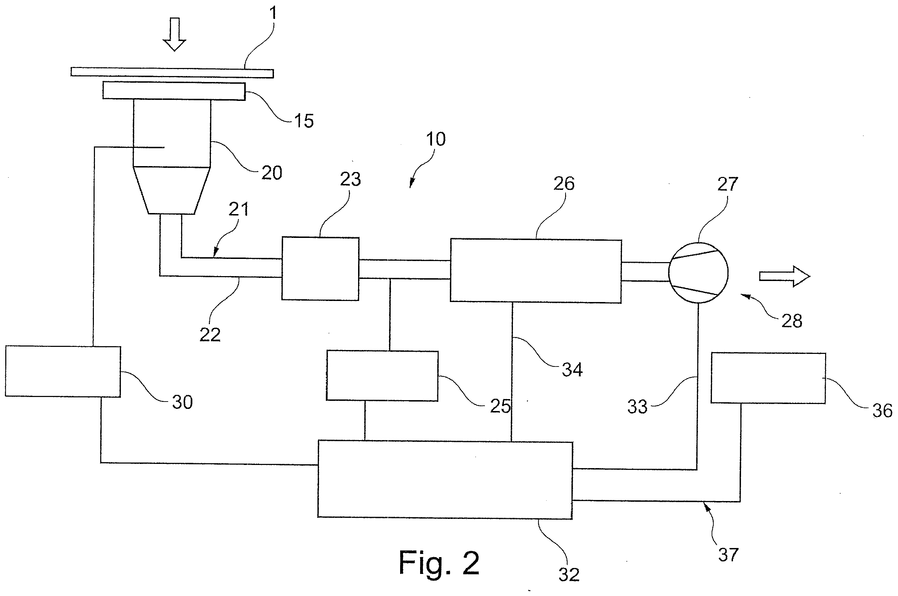

In der Fig. 1 ist eine Vorrichtung 10 zur Prüfung der Luftdurchlässigkeit von Sporttextilien, insbesondere zur Prüfung der Luftdurchlässigkeit einer Textilprobe 1 (s. Fig. 2) dargestellt.

Bei der Textilprobe 1 handelt es sich insbesondere um einen Teil eines Skisprunganzuges oder eines Schwimmanzuges. Mittels der Vorrichtung 10 soll die durch Wettkampfregeln bzw. Spezifikationen festgelegte Luftdurchlässigkeit der Textilprobe 1, insbesondere deren minimale Luftdurchlässigkeit, überprüft werden. Insbesondere ist ein Einsatz der Vorrichtung 10 am Wettkampfort bzw. am Ort der Sportveranstaltung vorgesehen. Hierzu weist die Vorrichtung 10 einen in der Fig. 1 dargestellten, tragbaren Messkoffer 11 auf, in dem alle Einrichtungen zur Überprüfung der Luftdurchlässigkeit der Textilprobe 1 integriert bzw. angeordnet sind. Zum Transport bzw. zum Schutz der Vorrichtung 10 kann der Messkoffer 11 mittels eines in der Fig. 1 nicht dargestellten Deckelelements überdeckt bzw. abgeschlossen werden.

Der Messkoffer 11 weist an seiner Oberseite 12 insbesondere wenigstens einen Einstellknopf 13 und ein Display 14 auf. Über den Einstellknopf 13 kann beispielsweise ein bestimmter Wert (z.B. ein bestimmter Differenzdruck oder ein Wert zur Feinabstimmung) eingestellt werden.

Mittels des Displays 14 wird z.B. der aktuelle Status bzw. das Prüfergebnis dargestellt.

Ferner erkennt man an der Oberseite 12 des Messkoffers 11 eine im Ausführungsbeispiel ringförmige Aufnahme 15, auf die die in der Fig. 2 dargestellte Textilprobe 1 aufgelegt werden kann. Die Textilprobe 1 lässt sich mittels einer Spanneinrichtung 16, die z.B. einen Spannring 17 umfasst, auf der Aufnahme 15 positionieren bzw. auf der Aufnahme 15 fixieren. Vorteilhafterweise weist die Spanneinrichtung 16, die über einen Spannhebel 18 an der Oberseite 12 des Messkoffers 11 schwenkbar angeordnet ist, eine im Näheren nicht dargestellte Einrichtung zum Erkennen des korrekten bzw. vollständigen Einspannens der Textilprobe 1 an der Aufnahme 15 auf.

In der Fig. 2 ist der wesentliche Aufbau der Vorrichtung 10 bzw. der Inhalt des Messkoffers 11 schematisch dargestellt. Hierbei weist die Vorrichtung 10 eine Meßkammer 20 auf, die Bestandteil eines Luftführungs- und Messgehäuses 21 ist. Das Luftführungs- und Messgehäuse 21 weist einen in der Fig. 2 lediglich angedeuteten Luftführungskanal 22 auf, in dem ein Filterelement 23, zumindest ein Messfühler eines Temperatursensors 25, ein Luftmengensensor 26 und eine Gebläseeinrichtung 27 integral angeordnet sind. Mittels der Gebläseeinrichtung 27 wird Umgebungsluft unter Durchströmung der in der Aufnahme 15 eingespannten Textilprobe 1 angesaugt, durchströmt die Meßkammer 20, das Filterelement 23 und den Luftmengensensor 26 und wird an einem Luftaustritt 28 aus der Vorrichtung 10 ausgeleitet.

Die Meßkammer 20 ist in Wirkverbindung mit einem Differenzdrucksensor 30 angeordnet, der einerseits den Druck innerhalb der Meßkammer 20 und andererseits den Umgebungsdruck erfasst. Dabei wird das Differenzsignal zwischen dem Umgebungsdruck und dem kleineren Druck innerhalb der Meßkammer 20 einer Steuereinrichtung 32 der Vorrichtung 10 als Eingangswert zugeführt.

Die Steuereinrichtung 32 umfasst vorzugsweise einen Mikrocomputer mit Auswertemitteln, die insbesondere einen Auswertealgorithmus zur Berechnung der Luftdurchlässigkeit der Textilprobe 1 umfassen, dessen Ergebnis auf dem Display 14 angezeigt wird, sowie eine Druckregelung, welche durch Ansteuerung der Gebläseeinrichtung 27 über eine Steuerleitung 33 erfolgt, um einen bestimmten Differenzdruck einzustellen.

Der zwischen dem Filterelement 23 und dem Luftmengensensor 26 angeordnete Temperatursensor 25, welcher bevorzugt möglichst nahe vor dem Luftmengensensor 26 angeordnet ist, führt den erfassten Wert der Lufttemperatur ebenfalls der Steuereinrichtung 32 als Eingangsgröße zu. Ebenso ist der Luftmengensensor 26, welcher beispielsweise ein vom Luftstrom angetriebenes Lüfterrad aufweist, über eine Leitung 34 mit der Steuereinrichtung 32 gekoppelt und führt z.B. einen in Abhängigkeit von der Strömungsgeschwindigkeit abhängigen Wert des Luftdurchsatzes der Steuereinrichtung 32 als Eingangsgröße zu.

Wesentlich ist, dass die Steuereinrichtung 32 zusätzlich mit einem Absolutdrucksensor 36 gekoppelt ist, der den Luftdruck der Umgebungsluft misst und über eine Leitung 37 der Steuereinrichtung 32 als zusätzlichen Eingangswert zur Kompensation des Aufstellungsortes des Meßkoffers 11 bzw. des Meßortes zuführt.

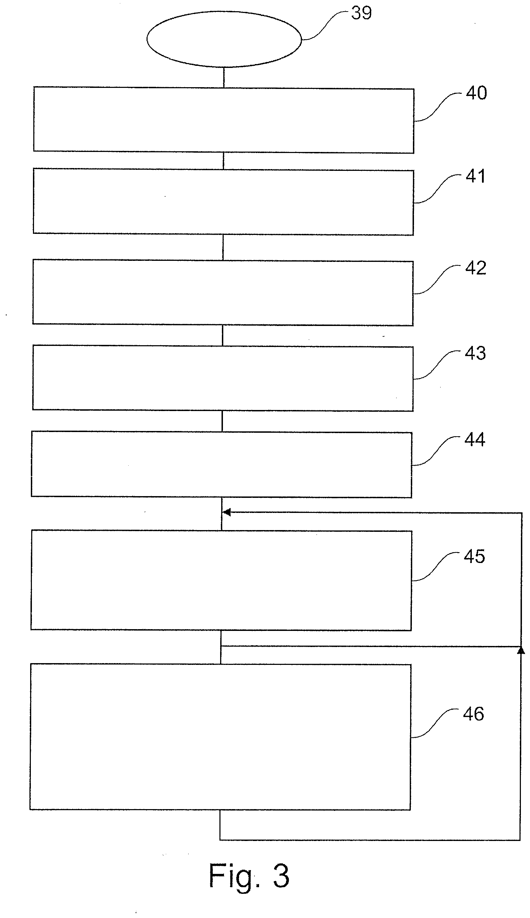

In der Fig. 3 ist beispielhaft ein vollautomatisierter Prüfablauf mittels der Vorrichtung 10 dargestellt. Dieser Prüfablauf umfasst nach Betätigen beispielsweise eines Startknopfes in einem ersten Programmschritt 39 das Laden von Geräteparametern in einem zweiten Programmschritt 40. Anschließend findet in einem dritten Programmschritt 41 ein Testlauf der Gebläseeinrichtung 27 statt. Danach erfolgt in einem vierten Programmschritt 42 ein Selbsttest, der beispielsweise das Prüfen der Sensoren (Temperatursensor 25, Luftmengensensor 26, Differenzdrucksensor 30 und Absolutdrucksensor 36) auf innerhalb bestimmter Grenzen liegenden Eingangssignale testet, um damit auf eine korrekte Funktion bzw. einen korrekten Anschluss der Sensoren zu schließen.

In einem nachfolgenden fünften Programmschritt 43 findet eine Temperaturstabilisierung der Vorrichtung 10 statt. Das bedeutet, dass die Vorrichtung 10 in dem fünften Programmschritt 43 solange betrieben wird, bis innerhalb des Luftführungs- und Messgehäuses 21 eine nahezu konstante Temperatur herrscht, welche mittels des Temperatursensors 25 erfasst wird.

Anschließend findet in einem sechsten Programmschritt 44 die Tarierung der Vorrichtung 10 statt. Diese Tarierung erfolgt vorteilhafterweise, jedoch nicht einschränkend dadurch, dass in die Aufnahme 15 eine (in den Figuren nicht dargestellte) Messblende mit einem bestimmten Luftdurchsatz zur Simulation einer Textilprobe 1 eingesetzt wird, deren bekannter Luftdurchsatz mit der Vorrichtung 10 nachgemessen wird. Hierbei findet bevorzugt eine Messblende Verwendung, deren bekannter Luftdurchsatz möglichst nahe an dem zu überprüfenden Grenzwert der Textilprobe 1 gemäß Wettkampf- bzw. Bekleidungsnorm liegt. Dadurch ergibt sich der Vorteil, dass die Vorrichtung 10 bei der Überprüfung des Luftdurchsatzes der tatsächlichen Textilprobe 1 im Bereich des Grenzwertes eine besonders hohe Messgenauigkeit aufweist. Der von der Vorrichtung 10 für die Messblende erfasste Wert des Luftdurchsatzes wird über das Display 14 angezeigt. Mittels beispielsweise des Einstellknopfes 13 wird anschließend bei einer Abweichung des angezeigten Messwertes von dem bekannten Wert für den Luftdurchsatz der Messblende die Vorrichtung 10 bzw. die Steuereinrichtung 32 auf den Wert der Messblende eingestellt. Sobald dies geschehen ist, ist die Vorrichtung 10 zum eigentlichen Prüfbetrieb bereit.

Daher wird in einem siebten, als Dauerschleife ausgebildeten Programmschritt 45 solange gewartet, bis die Textilprobe 1 in der Aufnahme 15 eingespannt ist, was insbesondere über die (verrastete) Position des Spannhebels 18 festgestellt werden kann. Sobald dies geschehen ist, geht das Programm in einen achten Programmschritt 46 über, der eigentlichen Messung des Luftdurchsatzes der Textilprobe 1 mit einem Hochfahren der Gebläseeinrichtung 27 und eine Regelung des Differenzdrucks in der Meßkammer 20 im Verhältnis zum Umgebungsdruck umfasst. Hierbei werden bei einem geregelten Differenzdruck die Messwerte des Luftsensors 26 , des Temperatursensors 25 und des Absolutdrucksensors 36 der Steuereinrichtung 32 als Eingangsgrößen zugeführt, welche anhand des Auswertungsalgorithmus einen entsprechenden Wert für die Luftdurchströmung berechnet und an das Display 14 als Ausgangswert ausgibt.

Hierbei können beispielsweise für verschiedenste, vom Temperatursensor 25 und Absolutdrucksensor 36 erfasste Messwerte in der Steuereinrichtung 32 Kompensations- bzw. Korrekturfaktoren in tabellarischer Form oder in Kurvenform als Gleichung abgelegt sein, mit deren Hilfe der Wert für die Luftdurchströmung korrigiert wird. Die Kompensations- bzw. Korrekturfaktoren sind wiederum beispielweise anhand von Eichkurven ermittelt worden.

Das Beenden der Messung kann entweder durch das Drücken eines entsprechenden Betätigungsknopfes, oder z.B. durch Öffnen des Spannhebels 18 erfolgen, wodurch die Gebläseeinrichtung 27 abgestellt wird.

Die soweit beschriebene Vorrichtung 10 zur Prüfung der Luftdurchlässigkeit von Sporttextilien kann in vielfältiger Art und Weise abgewandelt bzw. modifiziert werden, ohne vom Erfindungsgedanken abzuweichen. Dieser besteht in der Verwendung eines zusätzlichen Absolutdrucksensors 36 zur Erfassung des Umgebungsluftdruckes, um örtliche Gegebenheiten des Aufstellungsorts der Vorrichtung 10 zu erfassen und in der Bewertung bzw. Auswertung der Luftdurchlässigkeit der Textilprobe 1 zu berücksichtigen.

### Bezugszeichenliste

- 1: Textilprobe

- 10: Vorrichtung
- 11: Messkoffer
- 12: Oberseite
- 13: Einstellknopf
- 14: Display
- 15: Aufnahme
- 16: Spanneinrichtung
- 17: Spannring
- 18: Spannhebel

- 20: Druckkammer
- 21: Luftführungs- und Messgehäuse
- 22: Luftführungskanal
- 23: Filterelement

- 25: Temperatursensor
- 26: Luftmengensensor
- 27: Gebläseeinrichtung
- 28: Luftaustritt
- 29: Gebläseeinrichtung
- 30: Differenzdrucksensor

- 32: Steuereinrichtung
- 33: Steuerleitung
- 34: Leitung

- 36: Absolutdruck
- 37: Leitung

- 39: 1. Schritt
- 40: 2. Schritt
- 41: 3. Schritt
- 42: 4. Schritt
- 43: 5. Schritt
- 44: 6. Schritt
- 45: 7.Schritt
- 46: 8. Schritt

## Patentansprüche

1. Vorrichtung (10) zur Prüfung der Luftdurchlässigkeit von Sporttextilien, mit einem Messgehäuse (21), das eine Aufnahme (15) für eine insbesondere flächige Textilprobe (1) aufweist, einer Gebläseeinrichtung (27) zur Erzeugung insbesondere eines Unterdrucks, wobei die Gebläseeinrichtung (27) mit dem Messgehäuse (21) derart gekoppelt ist, dass Luft unter Durchströmung der Textilprobe (1) das Messgehäuse (21) durchströmt, einer ersten Einrichtung (30) zur Erfassung des Differenzdrucks zwischen den beiden Seiten der Textilprobe (1), einer zweiten Einrichtung (26) zur Erfassung des Luftvolumenstroms in dem Messgehäuse (21) und Auswertemitteln (32) zum Berechnen des Luftdurchsatzes durch die Textilprobe (1) unter Berücksichtigung von Eingangswerten der ersten und zweiten Einrichtung (26, 30),
**dadurch gekennzeichnet,**
**dass** die Vorrichtung zum mobilen Einsatz an einer Weftkampfstelle ausgebildet ist,
**dass** Auswertemittel (32) mit einer Kompensationseinrichtung (25, 36) zur Erfassung des Absolutdrucks der Umgebungsluft als Umgebungsbedingungen des Ortes der Vorrichtung (10) gekoppelt sind und, dass die Auswertemittel (32) den Luftdurchsatz durch die Textilprobe (1) unter Berücksichtigung der von der Kompensationseinrichtung (25, 36) erfassten Messwerte berechnen.

2. Vorrichtung nach Anspruch 1,
**dadurch gekennzeichnet,**
**dass** die Kompensationseinrichtung zusätzlich eine Einrichtung (25) zur Erfassung der Temperatur der durch die Textilprobe (1) durchströmenden Luft aufweist.

3. Vorrichtung nach Anspruch 2,
**dadurch gekennzeichnet,**
**dass** die Einrichtung zur Erfassung (25) der Temperatur im Bereich der zweiten Einrichtung (26) angeordnet ist.

4. Vorrichtung nach einem der Ansprüche 1 bis 3,
**dadurch gekennzeichnet,**
**dass** die Vorrichtung (10) als tragbare Prüfeinrichtung mit einem vorzugsweise einteiligen Prüfstandskoffer (11) ausgebildet ist, der ggf. mit einem Schutzelement zum Abdecken des Prüfstandskoffers (11) verbindbar ist.

5. Verfahren zur Prüfung der Luftdurchlässigkeit von Sporttextilien, insbesondere mittels einer Vorrichtung (10) nach einem der Ansprüche 1 bis 4, bei dem durch ein Messgehäuse (21) Umgebungsluft unter Durchströmung einer Textilprobe (1) angesaugt wird, wobei zwischen dem Umgebungsluftdruck und dem Druck im Messgehäuse (21) ein Differenzdruck erfasst wird, wobei der durch das Messgehäuse (21) strömende Luftvolumenstrom gemessen wird und wobei der Messwert des Differenzdrucks und der Messwert des Luftvolumenstroms einer Auswerteeinrichtung (32) als Eingangsgrößen zugeführt werden, welche mittels eines Algorithmus einen Wert für die Luftdurchlässigkeit der Textilprobe (1) ermittelt,
**dadurch gekennzeichnet,**
**dass** der Auswerteeinrichtung (32) der Absolutdruck der Umgebungsluft als wenigstens eine zusätzliche Kompensationsgröße als Eingangswert zugeführt wird und, dass der Algorithmus den Wert für die Luftdurchlässigkeit der Textilprobe (1) unter Berücksichtigung der wenigstens einen zusätzlichen Kompensationsgröße ermittelt.

6. Verfahren nach Anspruch 5,
**dadurch gekennzeichnet,**
**dass** dem Messen der Luftdurchlässigkeit der Textilprobe (1) ein automatischer Prüfablauf zur Überprüfung von Komponenten und Sensoren vorausgeht.

7. Verfahren nach einem der Ansprüche 5 bis 6,
**dadurch gekennzeichnet,**
**dass** mittels eines Messelements mit bekanntem Luftdurchsatz die Auswerteeinrichtung (32) eingestellt wird und, dass der Luftdurchsatz des Messelements zumindest annähernd einem zulässigen Grenzwert für den Luftdurchsatz der Textilprobe (1) entspricht.

8. Verwendung einer Vorrichtung (10) zur Prüfung der Luftdurchlässigkeit von Textilien nach einem der Ansprüche 1 bis 4 zum Testen Skisprunganzügen und Schwimmanzügen.

## Claims

1. A device (10) for testing the air permeability of sports textiles, having a measuring housing (21), which comprises a receptacle (15) for an in particular planar textile sample (1), a blower means (27) for generating an underpressure, wherein the blower means (27) is coupled with the measuring housing (21) in such a way that air flows through the measuring housing (21) whilst flowing through the textile sample (1), a first device (30) for acquiring the differential pressure between the two sides of the textile sample (1), a second device (26) for acquiring the air volume flow in the measuring housing (21) and evaluation means (32) for calculating the air throughput through the textile sample (1) under consideration of input values of the first and second devices (26, 30),
**characterized in that**
the device is formed for mobile use at a competition site,
**in that** evaluation means (32) are coupled with a compensation device (25, 36) for detecting the absolute pressure of the environmental air as environmental conditions of the location of the device, and **in that** the evaluation means (32) calculate the air throughput through the textile sample (1) under consideration of the measuring values detected by the compensation device (25, 36).

2. The device according to claim 1,
**characterized in that**
the compensation device additionally comprises a device (25) for detecting the temperature of the air flowing through the textile sample (1).

3. The device according to claim 2,
**characterized in that**
the device for detecting (25) the temperature is arranged in the area of the second device (26).

4. The device according to one of claims 1 to 3,
**characterized in that**
the device (10) is formed as a portable test device with a preferably integral testbed case (11), which, if required, is connectable to a protective element for covering the testbed case (11).

5. A method for testing the air permeability of sports textiles, in particular by means of a device (10) according to one of claims 1 to 4, in which environmental air is suctioned by a measuring housing (21) whilst flowing through a textile sample (1), wherein a differential pressure between the environmental air pressure and the pressure in the measuring housing (21) is detected, wherein the air volume flow flowing through the measuring housing (21) is measured and wherein the measured value of the differential pressure and the measured value of the air volume flow are supplied as input variables to an evaluation device (32), which determines a value for the air permeability of the textile sample (1) by means of an algorithm,
**characterized in that**
the absolute pressure of the environmental air is supplied to the evaluation device (32) as at least one additional compensation value as input variable, and **in that** the algorithm determines the value for the air permeability of the textile sample (1) under consideration of the at least one additional compensation value.

6. The method according to claim 5,
**characterized in that**
an automatic test procedure for testing components and sensors is carried out prior to the measurement of the air permeability of the textile sample (1).

7. The method according to one of claims 5 to 6,
the evaluation device (32) is set by means of a measuring element having a known air throughput, and in that the air throughput of the measuring element corresponds at least approximately to an allowable threshold value for the air throughput of the textile sample (1).

8. Use of a device (10) for testing the air permeability of textiles according to one of claims 1 to 4 for testing ski jumpsuits and swimsuits.

## Revendications

1. Dispositif (10) de contrôle de la perméabilité à l'air de textiles pour le sport, comprenant un boîtier de mesure (21) qui possède un logement (15) pour un échantillon de textile (1) notamment plat, un appareil de soufflage (27) destiné à produire notamment une dépression, l'appareil de soufflage (27) étant accouplé au boîtier de mesure (21) de telle sorte qu'un flux d'air passe à travers le boîtier de mesure (21) en passant à travers l'échantillon de textile (1), un premier appareil (30) destiné à détecter la pression différentielle entre les deux côtés de l'échantillon de textile (1), un deuxième appareil (26) destiné à détecter le débit volumique d'air dans le boîtier de mesure (21) et des moyens d'interprétation (32) destinés à calculer le débit d'air à travers l'échantillon de textile (1) en tenant compte des valeurs d'entrée du premier et du deuxième appareil (26, 30), **caractérisé en ce que** le dispositif est configuré pour une utilisation mobile sur un site de compétition, que les moyens d'interprétation (32) sont couplés à un appareil de compensation (25, 36) destiné à détecter la pression absolue de l'air ambiant en tant que conditions environnantes du lieu du dispositif (10) et que les moyens d'interprétation (32) calculent le débit d'air à travers l'échantillon de textile (1) en tenant compte des valeurs mesurées par l'appareil de compensation (25, 36).

2. Dispositif selon la revendication 1, **caractérisé en ce que** l'appareil de compensation possède en plus un appareil (25) destiné à détecter la température de l'air qui passe à travers l'échantillon de textile (1).

3. Dispositif selon la revendication 2, **caractérisé en ce que** l'appareil (25) destiné à détecter la température de l'air est disposé dans la zone du deuxième appareil (26).

4. Dispositif selon l'une des revendications 1 à 3, **caractérisé en ce que** le dispositif (10) est réalisé sous la forme d'un appareil de contrôle portable comprenant une mallette de banc d'essai (11) de préférence d'une seule pièce, lequel peut être éventuellement relié à un élément de protection destiné à couvrir la mallette de banc d'essai (11).

5. Procédé de contrôle de la perméabilité à l'air de textiles pour le sport, notamment au moyen d'un dispositif (10) selon l'une des revendications 1 à 4, avec lequel de l'air ambiant est aspiré à travers un boîtier de mesure (21) en traversant un échantillon de textile (1), une pression différentielle entre la pression de l'air ambiant et la pression dans le boîtier de mesure (21) étant détectée, le débit volumique d'air qui s'écoule à travers le boîtier de mesure (21) étant mesuré et la valeur mesurée du débit volumique d'air étant acheminée en tant que grandeur d'entrée à un appareil d'interprétation (32) qui, au moyen d'un algorithme, détermine une valeur pour la perméabilité à l'air de l'échantillon de textile (1), **caractérisé en ce**
**que** la pression absolue de l'air ambiant est acheminée comme valeur d'entrée à l'appareil d'interprétation (32) en tant qu'au moins une grandeur de compensation supplémentaire, et que l'algorithme détermine la valeur pour la perméabilité à l'air de l'échantillon de textile (1) en tenant compte de l'au moins une grandeur de compensation supplémentaire.

6. Procédé selon la revendication 5, **caractérisé en ce qu'**une routine d'essai automatique destinée à contrôler les composants et les capteurs précède la mesure de la perméabilité à l'air de l'échantillon de textile (1).

7. Procédé selon les revendications 5 à 6, **caractérisé en ce que** l'appareil d'interprétation (32) est réglé au moyen d'un élément de mesure ayant un débit d'air connu et **en ce que** le débit d'air de l'élément de mesure correspond au moins approximativement à une valeur limite admissible pour le débit d'air de l'échantillon de textile (1).

8. Utilisation d'un dispositif (10) destiné au contrôle de la perméabilité à l'air de textiles selon l'une des revendications 1 à 4 pour tester des combinaisons de saut à ski et des combinaisons de natation.
